# EUROPEAN PATENT APPLICATION

(11) **EP 3 763 250 A1**
(43) Date of publication of application: **13.01.2021**
(21) Application number: 19763810.9
(22) Date of filing: 07.03.2019
(51) Int. Cl.: A47C 31/12, A47C 21/00, A47C 31/00, A61B 5/024, A61B 5/08, A61B 5/11, A61B 5/0476, A61B 5/01, G01W 1/02, G16H 20/00

(54) **SLEEPING ENVIRONMENT CONTROL DEVICE USING REINFORCEMENT LEARNING**

(30) Priority: 07.03.2018 KR 20180026666
(71) Applicant: ICBS Co., Ltd, Seoul 07803 (KR)
(72) Inventor: LEE, Jung Woo, Seoul 07665 (KR); LEE, Sang Jun, Seoul 08807 (KR); LEE, Sang Eun, Seoul 07665 (KR)
(74) Representative: Rondano, Davide
(86) International application number: PCT/KR2019/002635
(87) International publication number: WO 2019/172667

(57) **Abstract**

In an exemplary embodiment of the present disclosure, a sleeping environment control device using reinforcement learning is disclosed. The sleeping environment control device using reinforcement learning includes: a main body on which a user is located; a sensor unit configured to measure a biometric signal of the user and generate current state information and post-operation state information; an operation unit configured to control a temperature and humidity of the main body in order to change a sleeping state of the user based on a control signal of a processor; a processor including one or more cores; and a memory configured to store program codes executable in the processor, in which the processor may include: a sleeping adequacy information generation module which generates current sleeping adequacy information of the user based on the current state information and generates post-operation sleeping adequacy information of the user based on the post-operation state information; an operation information determination module which determines operation information controlling an operation of the operation unit by using an operation determination algorithm based on the current sleeping adequacy information; and an operation adequacy determination module which updates the operation determination algorithm by comparing the post-operation sleeping adequacy information with reference sleeping adequacy information and determining adequacy for the operation.

## Description

### [Technical Field]

The present disclosure relates to a sleeping environment control device, and more particularly, to a device for controlling a sleeping environment so as to improve sleep efficiency of a user.

### [Background Art]

Recently, as the quality of life has improved and interest in health has increased rapidly, interest in sleeping is increasing. There are many ways, such as exercise and diet, to maintain and improve health, but it is most important to manage sleep well, which takes up about 30% of the day. However, modern people are unable to get deep sleep due to stress, and suffer from sleep abnormalities, such as insomnia, excessive sleep, narcolepsy, and sleep apnea, and sleep disorders, such as nightmare, night cry, and sleepwalking.

Korean Patent No. 10-0791371 discloses, in order to overcome sleep abnormality and sleep disorder, a method of measuring a biometric signal of a user when the user sleeps, monitoring a sleeping state of the user through measurement information, analyzing monitoring information to improve sleep efficiency. However, the related art has a limitation in providing a high-quality sleeping environment because users individually have different sleeping patterns and have different degrees of the affection by the sleeping environment. Accordingly, there are demands in the industry for a sleep management solution that can improve sleep efficiency in response to a real-time sleeping pattern of an individual user.

### [Disclo sure]

### [Technical Problem]

The present disclosure is conceived in response to the foregoing background art, and to provide a sleeping environment control device using reinforcement learning for improving sleep efficiency of a user.

### [Technical Solution]

In order to solve the technical problem, an exemplary embodiment of the present disclosure discloses a sleeping environment control device using reinforcement learning including: a main body on which a user is located; a sensor unit configured to measure a biometric signal of the user and generate current state information and post-operation state information; an operation unit configured to control at least one of a temperature and humidity of the main body in order to change a sleeping state of the user based on a control signal of a processor; a processor including one or more cores; and a memory configured to store program codes executable in the processor, in which the processor may include: a sleeping adequacy information generation module which generates current sleeping adequacy information of the user based on the current state information and generates post-operation sleeping adequacy information of the user based on the post-operation state information; an operation information determination module which determines operation information controlling an operation of the operation unit by using an operation determination algorithm based on the current sleeping adequacy information; and an operation adequacy determination module which updates the operation determination algorithm by comparing the post-operation sleeping adequacy information with reference sleeping adequacy information and determining adequacy for the operation.

Alternatively, the sensor unit may include at least one of a user state measurement sensor which measures at least one of a heart rate, a respiration rate, movement, and brain waves of the user in a contact or non-contact manner, a temperature sensor which measures at least one of a temperature indoors in which the user sleeps and a body temperature of the user, and a humidity sensor which measures humidity indoors in which the user sleeps, and at least one of the current state information, indoor temperature information, and indoor humidity information may be obtained through at least one of the sensors.

Alternatively, the operation unit may be provided in the main body and supply at least one of hot wind and cold wind to the main body in order to control a body temperature of the user.

Alternatively, the current state information may include at least one of respiration state information, heart rate state information, brain wave information, and movement state information of the user measured in the sensor unit, and include a measurement result during one cycle of a sleep cycle rhythm of the user.

Alternatively, the current sleeping adequacy information may be generated based on the current state information measured during one cycle of a sleep cycle rhythm.

Alternatively, the post-operation state information may be generated based on a biometric signal during one cycle of a sleep cycle rhythm during which the operation unit performs an operation by a control signal of the processor.

Alternatively, the post-operation sleeping adequacy information may be generated based on the post-operation state information measured during one cycle of a sleep cycle rhythm.

Alternatively, the reference sleeping adequacy information may be generated based on a biometric signal that maximizes sleep efficiency of the user during one cycle of a sleep cycle rhythm and be a goal of the post-operation sleeping adequacy information.

Alternatively, the operation determination algorithm may be formed of an artificial neural network, and output a score of each of one or more pieces of candidate operation information by using the current sleeping adequacy information as an input, and determine the operation information based on the score of each candidate operation information.

Alternatively, the operation adequacy determination algorithm may determine adequacy for the operation by comparing the post-operation sleeping adequacy information during one cycle of the sleep cycle rhythm with the reference sleeping adequacy information and determining similarity, and update the operation determination algorithm so that a probability that the operation determination algorithm determines the operation information based on the current sleeping adequacy information increases when the similarity is high, or update the operation determination algorithm so that a probability that the operation determination algorithm determines the operation information based on the current sleeping adequacy information decreases when the similarity is low.

Another exemplary embodiment of the present disclosure discloses a method of controlling a sleeping environment by using reinforcement learning. The method may include: generating current state information by measuring a biometric signal; generating current sleeping adequacy information based on the current state information; determining operation information by using an operation determination algorithm based on the current sleeping adequacy information; generating post-operation state information by measuring a biometric signal after performing the environment control operation; generating post-operation state information by measuring a biometric signal of the user after performing the environment control operation; generating post-operation sleeping adequacy information based on the post-operation state information; determining adequacy for the environment control operation by comparing the post-operation sleeping adequacy information and reference sleeping adequacy information; and updating an operation determination algorithm based on the determination on the adequacy for the environment control operation.

Another exemplary embodiment of the present disclosure discloses a computer program which is executable by one or more processors and is stored in a computer readable medium. The computer program may cause the one or more processors to perform following operations, the operations including: an operation of generating current state information by measuring a biometric signal; an operation of generating current sleeping adequacy information based on the current state information; an operation of determining operation information by using an operation determination algorithm based on the current sleeping adequacy information; an operation of performing an environment control operation of a mattress main body based on the operation information; an operation of generating post-operation state information by measuring a biometric signal after performing the environment control operation; an operation of generating post-operation sleeping adequacy information based on the post-operation state information; an operation of determining adequacy for the environment control operation by comparing the post-operation sleeping adequacy information and reference sleeping adequacy information; and an operation of updating an operation determination algorithm based on the determination on the adequacy for the environment control operation.

### [Advantageous Effects]

The present disclosure may provide a sleeping environment control device using reinforcement learning for improving sleep efficiency of a user.

### [Description of Drawings]

Various aspects are described with reference to the drawings, and herein, like reference numerals are generally used to designate like constituent elements. In the exemplary embodiment below, for the purpose of description, a plurality of specific and detailed matters is suggested in order to provide general understanding of one or more aspects. However, it is apparent that the aspect(s) may be carried out without the specific and detailed matters.
FIG. 1 is a conceptual diagram illustrating a system of a sleeping environment control device using reinforcement learning according to an exemplary embodiment of the present disclosure.
FIG. 2 is a block diagram illustrating the sleeping environment control device using reinforcement learning according to the exemplary embodiment of the present disclosure.
FIG. 3 is a diagram illustrating an example of the sleeping environment control device using reinforcement learning according to the exemplary embodiment of the present disclosure.
FIG. 4 is a flowchart illustrating a sleeping environment control method using reinforcement learning according to an exemplary embodiment of the present disclosure.
FIG. 5 is a diagram illustrating an example of a sleep stage according to a sleep time of a general adult sleeping according to the exemplary embodiment of the present disclosure.
FIG. 6 is a diagram illustrating an example of a sleep stage change of a user according to a temperature control change of the sleeping environment control device using reinforcement learning according to the exemplary embodiment of the present disclosure.
FIG. 7 is a diagram illustrating an example of a sleep stage according to sleeping adequacy information after an operation and a sleep stage according to reference sleeping adequacy information according to the exemplary embodiment of the present disclosure.
FIG. 8 is a configuration diagram illustrating an artificial neural network configuring an operation determination algorithm according to the exemplary embodiment of the present disclosure.
FIG. 9 is a diagram illustrating an example of deriving a score through the operation determination algorithm according to the exemplary embodiment of the present disclosure.
FIG. 10 is a block diagram illustrating a means for implementing the sleeping environment control method using reinforcement learning according to the exemplary embodiment of the present disclosure.
FIG. 11 is a block diagram illustrating a module for implementing the sleeping environment control method using reinforcement learning according to the exemplary embodiment of the present disclosure.
FIG. 12 is a block diagram illustrating logic for implementing the sleeping environment control method using reinforcement learning according to the exemplary embodiment of the present disclosure.
FIG. 13 is a block diagram illustrating a circuit for implementing the sleeping environment control method using reinforcement learning according to the exemplary embodiment of the present disclosure.
FIG. 14 is a simple and general schematic diagram illustrating an example of a computing environment in which the exemplary embodiments of the present disclosure are implementable.

### [Best Mode]

Various exemplary embodiments will be described with reference to the drawings, and throughout the entire drawings, a similar reference numeral is used for indicating a similar constituent element. In the present specification, various descriptions are presented for understanding the present disclosure. However, it is apparent that the exemplary embodiments may be carried out even without the particular description. In other examples, publicly known structures and devices are provided in the form of a block diagram for easily describing the exemplary embodiments.

Terms, "component", "module", "system", and the like used in the present specification indicate a computer-related entity, hardware, firmware, software, a combination of software and hardware, or execution of software. For example, a component may be a procedure executed in a processor, a processor, an object, an execution thread, a program, and/or a computer, but is not limited thereto. For example, both an application executed in a computing device and the computing device may be components. One or more components may reside within a processor and/or an execution thread, and one component may be localized within one computer or may be distributed between two or more computers. Further, the components may be executed by various computer readable media having various data structures stored therein. For example, components may communicate through local and/or remote processing according to a signal (for example, data transmitted to another system through a network, such as Internet, through data and/or a signal from one component interacting with another component in a local system and a distributed system) having one or more data packets.

The descriptions of the presented exemplary embodiments are provided so as for those skilled in the art to use or carry out the present disclosure. Various modifications of the exemplary embodiments may be apparent to those skilled in the art, and general principles defined herein may be applied to other exemplary embodiments without departing from the scope of the present disclosure. Accordingly, the present disclosure is not limited to the exemplary embodiments suggested herein, and shall be interpreted within the broadest meaning range consistent to the principles and new characteristics suggested herein.

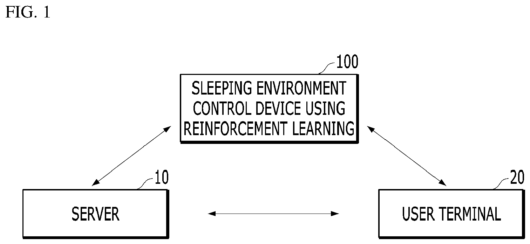

FIG. 1 is a conceptual diagram illustrating a system of a sleeping environment control device 100 using reinforcement learning according to an exemplary embodiment of the present disclosure.

According to the exemplary embodiment of the present disclosure, the sleeping environment control device 100 using reinforcement learning, a server 10, and a user terminal 20 may transmit and receive information through wireless and/or wired interconnection.

According to the exemplary embodiment of the present disclosure, the sleeping environment control device 100 using reinforcement learning may measure and determine a sleeping state of a user and control a sleeping environment of the user in response to the determined sleeping state. More particularly, the sleeping environment control device 100 using reinforcement learning may measure a sleeping state of the user. Further, the sleeping environment control device 100 using reinforcement learning may generate information on a sleeping state through the measured information of the user and determine a quality of the sleep. The sleeping environment control device 100 using reinforcement learning may provide the user with a sleeping environment that improves sleep efficiency through the determination on the quality of the sleep of the user.

According to the exemplary embodiment of the present disclosure, the sleeping environment control device 100 using reinforcement learning may perform reinforcement learning for a sleeping environment control operation. In particular, the sleeping environment control device 100 using reinforcement learning may recognize a current sleeping state of the user in the sleeping environment of the user. Further, the sleeping environment control device 100 using reinforcement learning may select and learn an operation or an operation order that maximizes a compensation among the selectable operations according to the recognized sleeping state of the user. Accordingly, the sleeping environment control device 100 using reinforcement learning may determine an operation method of an operation unit 120 through the learning, and may change the sleeping environment of the user through the control of the sleeping environment through the operation unit 120 and improve sleep efficiency of the user.

According to the exemplary embodiment of the present disclosure, the sleeping environment control device 100 using reinforcement learning may measure and determine a sleeping pattern and a quality of sleep without specialized knowledge or training on the analysis and the determination on the sleeping state of the user. The sleeping environment control device 100 using reinforcement learning may measure a biometric signal of the user and monitor the sleeping state of the user through a feedback loop, and determine a quality of sleep. Further, the sleeping environment control device 100 using reinforcement learning may improve the sleeping environment through the determination on the quality of sleep of the user and improve sleep efficiency of the user. Accordingly, the sleeping environment control device 100 using reinforcement learning may provide an optimum sleeping environment for each user sleeping state.

According to another exemplary embodiment of the present disclosure, the server 10 may control a sleeping environment control operation of the sleeping environment control device 100 using reinforcement learning by performing reinforcement learning. In particular, the server 10 may receive data related to the sleep of a user from the sleeping environment control device 100 using reinforcement learning and perform reinforcement learning. In detail, the server 10 may measure a sleeping state of the user and determine the sleeping state of the user based on the measured information. Further, the server 10 may transmit a control signal for controlling a sleeping environment to at least one of the sleeping environment control device 100 using reinforcement learning and the user terminal 20 through the determination on the sleeping state of the user to improve the sleeping environment of the user and improve sleep efficiency of the user. That is, the server 10 may perform reinforcement learning in order to provide an optimum sleeping environment for a sleeping state of each user.

According to the exemplary embodiment of the present disclosure, the user terminal 20 may transmit user information to at least one of the sleeping environment control device 100 using reinforcement learning and the server 10.

According to the exemplary embodiment of the present disclosure, the user terminal 20 may control the environment control operation of the sleeping environment control device 100 using reinforcement learning. In particular, the user terminal 20 may transmit control information to the sleeping environment control device 100 using reinforcement learning. In this case, the control information may be generated based on a user's input or generated based on the reinforcement learning performed by the server 10. Otherwise, the control information may include an operation range of a temperature and humidity operated by the sleeping environment control device 100 using reinforcement learning for controlling the sleeping environment. That is, the user terminal 20 may transmit the control information to the sleeping environment control device 100 using reinforcement learning based on at least one of the user's input and the reinforcement learning performed by the server 10.

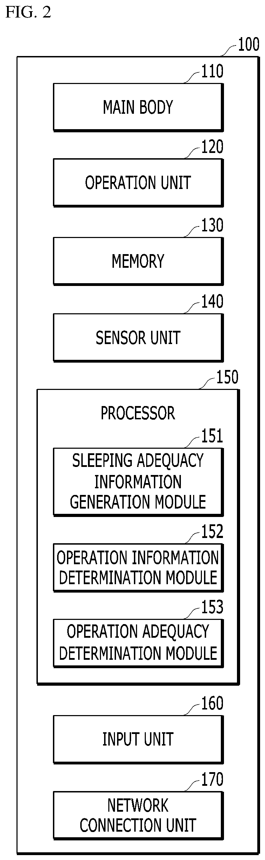

FIG. 2 is a block diagram illustrating the sleeping environment control device 100 using reinforcement learning according to the exemplary embodiment of the present disclosure.

The components of the sleeping environment control device 100 using reinforcement learning illustrated in FIG. 2 are illustrative. Only some of the components may also configure the sleeping environment control device 100 using reinforcement learning. Further, in addition to the components, additional components may be included in the sleeping environment control device 100 using reinforcement learning.

A method of controlling a sleeping environment, which improves sleep efficiency of a user, by the sleeping environment control device 100 using reinforcement learning according to an exemplary embodiment of the present disclosure will be described.

According to the exemplary embodiment of the present disclosure, the sleeping environment control device 100 using reinforcement learning may maximize sleep efficiency of a user. In particular, the sleeping environment control device 100 using reinforcement learning may determine a sleeping state of a user and control a sleeping environment to improve sleep efficiency of the user. In detail, the sleeping environment control device 100 using reinforcement learning may learn a sleeping environment control operation by the reinforcement learning method with a processor 150. The sleeping environment control device 100 using reinforcement learning may determine an operation method of the operation unit 120 through the learning. The operation unit 120 operating through the learning may operate so as to adjust a temperature and humidity of a main body 110 for changing a sleeping state of the user. Accordingly, the sleeping environment control device 100 using reinforcement learning may provide the user with an optimum sleeping environment through the sleeping environment control operation of the operation unit 120.

As illustrated in FIG. 2, the sleeping environment control device 100 using reinforcement learning may include the main body 110, the operation unit 120, a memory 130, a sensor unit 140, the processor 150, an input unit 160, and a network connection unit 170.

According to the exemplary embodiment of the present disclosure, the sleeping environment control device 100 using reinforcement learning may be configured as a predetermined device that allows the user to sleep, for example, a bed, a soil bed, a stone bed, an electric mat, a hot water mat, and a cold water mat, to control a sleeping environment of the user. The particular description of the sleeping environment control device 100 using reinforcement learning is merely illustrative, and the present disclosure is not limited thereto.

According to the exemplary embodiment of the present disclosure, the main body 110 is a member on which a body of the user is laid, and which is substantially in contact with the body of the user and supports the body of the user to provide a sleeping place, and may include any configuration in which the user may lie down or lean on while feeling comfortable. The main body 110 may be one of the bedclothes, such as a bed mattress, a topper, a pad, a floor covering, and a blanket, and is limited thereto.

According to the exemplary embodiment of the present disclosure, an internal passage 111 may be provided at an internal side of the main body 110. The internal passage 111 may be a space in which hot wind or cold wind supplied from the operation unit 120 moves. The hot wind and cold wind provided form the operation unit 120 may pass through an upper end of the internal passage 111 to adjust a temperature of the main body 110. Further, the temperature of the hot wind and cold wind passing through the upper end of the internal passage 111 is maintained by bedclothes 112 (for example, a blanket). That is, the sleeping environment control device 100 using reinforcement learning may bring a high-efficiency sleeping environment change with a minimal operation.

According to the exemplary embodiment of the present disclosure, the operation unit 120 may adjust at least one of a temperature and humidity of the main body 110. For example, the operation unit 120 may supply at least one of hot wind and cold wind to the main body 110 supporting the user in order to control a body temperature of the user. Further, the operation unit 120 may perform at least one of a dehumidification operation and a humidification operation so as to control humidity of the main body 110 supporting the user. Accordingly, the operation unit 120 may improve sleep efficiency by adjusting a body temperature and humidity of the user.

According to the exemplary embodiment of the present disclosure, the operation unit 120 may include at least one of a cooler, a warmer, a humidifier, and a dehumidifier. Further, the operation unit 120 may be a device using electricity or hot water. For example, the operation unit 120 may include an electric mat, a hot water mat, and a cold water mat. The electric mat may include a heating cloth having a heating wire so as to generate heat by supplying electricity to at least one surface of an upper portion of a predetermined mattress. The hot water mat or cold water mat has a predetermined hot water or cold water circulating device so as for hot water or cold water to be circulated to provide a predetermined temperature. That is, any device that provides an environment suitable to the sleep of the user by controlling a temperature and humidity is applicable as the operation unit 120. The configurations provided in the operation unit 120 are merely illustrative, and the present disclosure is not limited thereto.

According to the exemplary embodiment of the present disclosure, the environment control operation of the operation unit 120 may be based on control information received from the processor 150. In particular, the operation unit 120 may receive a control signal from the processor 150. Herein, the control signal may be a signal determined by an operation information determination module 152. Further, the control signal may include at least one of a temperature adjusting operation and a humidity adjusting operation to be operated by the operation unit 120, and an operation time of each operation.

According to another exemplary embodiment, the environment adjusting operation of the operation unit 120 may be based on control information received from at least one of the server 10 and the user terminal 20. More particularly, the operation unit 120 may receive control information from at least one of the server 10 and the user terminal 20. Herein, the control information may be generated based on a user's input or generated based on the reinforcement learning performed by the server 10. Further, the control information may include at least one of a temperature adjusting operation and a humidity adjusting operation to be operated by the operation unit 120, and an operation time of each operation. That is, the sleeping environment control device 100 using reinforcement learning may control the sleeping environment control operation of the operation unit 120 based on the input of the user.

That is, the operation unit 120 may perform the sleeping environment control operation through the control information generated from the server and the user terminal, in addition to the control signal of the processor 150.

According to the exemplary embodiment of the present disclosure, the sensor unit 140 may include a user state measurement sensor which measures at least one of a heart rate, a respiration rate, movement, and brain waves of the user in a contact or non-contact manner. The sensor unit 140 may measure a biometric signal of the user by using the user state measurement sensor. The sensor unit 140 may measure at least one of a respiration signal for respiration of the user, a heart rate signal for a heartbeat of the user, and a movement signal for a movement of the user through the user state measurement sensor that measures a size and frequency of a pressure applied by the user.

Further, the sensor unit 140 may include at least one of a temperature sensor that measures at least one of a temperature indoor in which the user sleeps and a body temperature of the user, and a humidity sensor that measures humidity indoors in which the user sleeps.

According to the exemplary embodiment of the present disclosure, the sensor unit 140 may additionally include various sensors useful for analyzing sleeping. For example, sensors, such as an acoustic sensor, a brainwave measurement sensor, a blood pressure manometer, and an air quality sensor, may be used, and the present disclosure is not limited thereto.

According to the exemplary embodiment of the present disclosure, the sensor unit 140 may be located at any place at which the biometric signal of the user may be measured, and may also be located while being separated from the sleeping environment control device 100 using reinforcement learning.

According to the exemplary embodiment of the present disclosure, the processor 150 may control the operation unit 120 which adjusts the sleeping environment for changing the sleeping state of the user. In particular, the processor 150 may transmit the control signal to the operation unit 120. In detail, the control signal may be the signal that causes the operation unit 120 to perform at least one of a hot air supply operation, a cold air supply operation, a dehumidification operation, and a humidification operation. For example, when the sleeping environment control device 100 using reinforcement learning wants to lower a body temperature of the user and increase humidity of a space in which the user is located, the control signal may be the signal that causes the operation unit 120 to perform the cold air supply operation and the humidification operation, and the operation unit 120 may perform the cold air supply operation and the humidification operation to lower the body temperature of the user and increase humidity of the space in which the user is located. That is, the control signal may control the operation unit so as to change various conditions of the sleeping environment to influence the quality of sleep of the user and enable the user to take sleep in the improved environment. The processor 150, which generates the control signal controlling the operation of the operation unit 120 may include a sleeping adequacy information generation module 151, an operation information determination module 152, and an operation adequacy determination module 153. The modules may generate control signals controlling the operation of the operation unit 120 by the following methods.

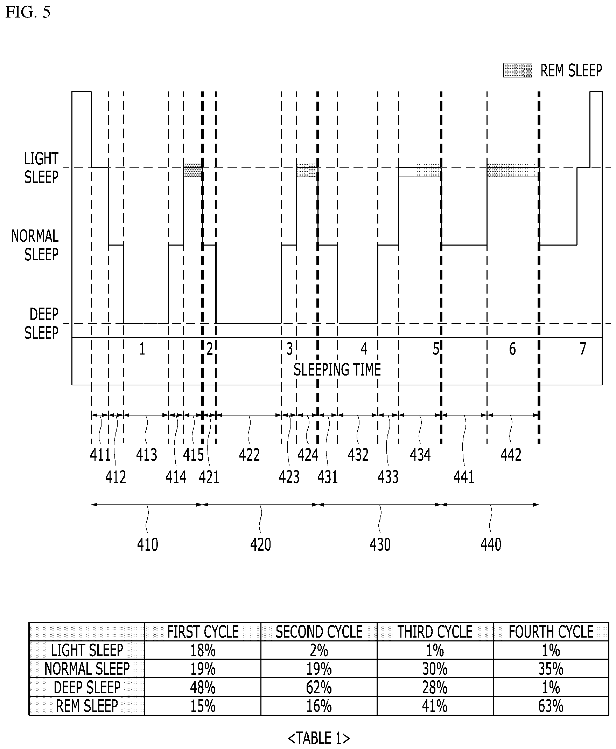

According to the exemplary embodiment of the present disclosure, the sleeping adequacy information generation module 151 may generate current sleeping adequacy information of the user based on the current state information. In detail, the sleeping adequacy information generation module 151 may generate current sleeping adequacy information based on the current state information generated based on the measured information measured through the sensor unit 140. In this case, the current sleeping adequacy information may be generated in response to the current state information measured during one cycle of a sleep cycle rhythm. Accordingly, the current sleeping adequacy information may be formed of information on the user's sleeping state during one cycle of the sleep cycle rhythm. For example, the current sleeping adequacy information may include a ratio information representing a ratio of each sleep stage during one cycle. In this case, each sleep stage may be formed of a light sleep stage, a normal sleep stage, a deep sleep stage, and a Rapid Eye Movement (REM) sleep stage. For example, FIG. 5 is a diagram illustrating an example of the sleep stage according to a sleeping time of a general adult sleeping according to the exemplary embodiment of the present disclosure. The sleeping environment control device 100 using reinforcement learning may determine one cycle of the sleep cycle rhythm of the user according to the sleep stage, and generate information based on the information during one cycle of the sleep cycle rhythm. The information generated based on the information during one cycle of the sleep cycle rhythm may include current sleeping adequacy information, post-operation sleeping adequacy information, and reference sleeping adequacy information.

As illustrated in FIG. 5, in general, an adult's sleep cycle may be repeated 4 to 5 times of a night. Further, as represented in Table 1 illustrated in FIG. 5, it can be seen that a ratio of the deep sleep is high in the early stage of sleep, and the ratio of REM sleep is high as the sleep goes to the later stage of sleep.

According to the exemplary embodiment of the present disclosure, reference numerals 410, 420, 430, 440 may represent one cycle of the sleep cycle rhythm. In particular, reference numeral 410 may be the first cycle of the sleep cycle rhythm of the user, reference numeral 420 may be the second cycle of the sleep cycle rhythm of the user, reference numeral 430 may be the third cycle of the sleep cycle rhythm of the user, and reference numeral 440 may be the fourth cycle of the sleep cycle rhythm of the user.

As illustrated in FIG. 5, the sleep stages during one cycle may have different ratios for each cycle. In detail, the first cycle 410 may be five sections including reference numerals 411, 412, 413, 414, and 415. The reference numerals 411, 412, 413, 414, and 415 may represent the change in the sleep stage according to the time during one cycle. Further, the second cycle 420 may be four sections including reference numerals 421, 422, 423, and 424. The reference numerals 421, 422, 423, and 424 may represent the change in the sleep stage according to the time during one cycle. Further, the third one cycle 430 may be four sections including reference numerals 431, 432, 433, and 434. The reference numerals 431, 432, 433, and 434 may represent the change in the sleep stage according to the time during one cycle. Further, the one fourth cycle 440 may be two sections including reference numerals 441 and 442. The reference numerals 441 and 442 may represent the change in the sleep stage according to the time during one cycle. That is, the degree of change in the sleep stage for each cycle is different, and the ratio thereof may also be different. For example, referring to Table 1 illustrated in FIG. 5, the ratios of light sleep, normal sleep, deep sleep, and REM sleep may be different between the first cycle 410 and the second cycle 420.

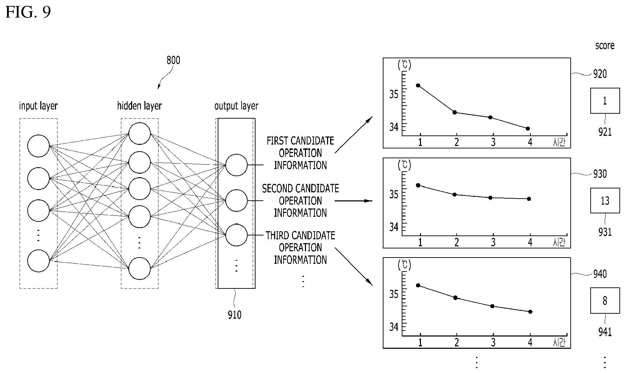

According to the exemplary embodiment of the present disclosure, the operation information determination module 152 may determine operation information generating the control signal controlling the operation of the operation unit 120 by using an operation determination algorithm based on the current sleeping adequacy information. The operation determination algorithm is formed of an artificial neural network, and may output a score of each of one or more pieces of candidate operation information by using the current sleeping adequacy information as an input. Further, the operation information determination module 152 may determine operation information based on the score of each of the candidate operation information. In particular, the operation determination algorithm may output a score of corresponding candidate operation information based on the current sleeping adequacy information on the sleeping state of the user during one cycle of the sleep cycle rhythm, and determine operation information having a maximum score among the candidate operation information. Herein, the candidate operation information is the operation information having a possibility of being the control signal, and may be one or more operations that cause the operation information determination module 152 to perform a specific operation. For example, as illustrated in FIG. 9, the score for each of the candidate operation information may be calculated through the artificial neural network 800 (reference numeral 910). In this case, three candidate operation information calculated through the artificial neural network 800 may be first candidate operation information, second candidate operation information, and third candidate operation information. The candidate operation information may include the first candidate operation information 920 that causes the operation information determination module 152 to lower the temperature to 34.6°C for one to two hours, the temperature to 34.3°C for two to three hours, and the temperature to 34°C for three to four hours, the second candidate operation information 930 that causes the operation information determination module 152 to lower the temperature to 35°C for one to two hours, the temperature to 34.8°C for two to three hours, and the temperature to 34.7°C for three to four hours, the third candidate operation information 940 that causes the operation information determination module 152 to lower the temperature to 34.8°C for one to two hours, the temperature to 34.6°C for two to three hours, and the temperature to 34.3°C for three to four hours, and the like. In this case, the operation determination algorithm may draw that a score of the first candidate operation information is 1 (reference numeral 921), a score of the second candidate operation information is 13 (reference numeral 931), and a score of the third candidate operation information is 8 (reference numeral 941) by processing the sleeping adequacy information through the artificial neural network 800. That is, the operation determination algorithm may determine the second candidate operation information as the operation information based on the score. Further, when the environment control operation is performed through the second candidate operation information among the candidate operations, sleep efficiency of the user may be most excellent. The candidate operation information, the number of candidate operation information, the number of nodes, the graph, and the scores illustrated in FIG. 9 are merely illustrative, and the number of candidate operation information and the score may be changed according to the sleeping environment of the user.

According to the exemplary embodiment of the present disclosure, the operation determination algorithm may be formed of the artificial neural network. The artificial neural network 800 may generally be formed of a set of mutually connected calculation units that may be called "nodes". The "nodes" may also be referred as "neurons". The neural network includes one or more nodes. The nodes (or neurons) configuring the neural network 200 may be interconnected by one or more "links".

In the neural network 800, one or more nodes connected through the links may relatively form a relationship with an input node and an output node. The concept of the input node is relative to the concept of the output node, and a predetermined node having an output node relationship with respect to one node may have an input node relationship in a relationship with another node, and a reverse relationship is also available. As described above, the relationship between the input node and the output node may be generated based on the link. One or more output nodes may be connected to one input node through a link, and a reverse case may also be valid.

In the relationship between an input node and an output node connected through one link, a value of the output node may be determined based on data input to the input node. Herein, a node connecting the input node and the output node may have a weight. The weight may be variable, and in order to perform a desired function of the neural network 800, the weight may be updated by a user or an algorithm. For example, when one or more input nodes are connected to one output node by links, respectively, a value of the output node may be determined based on values input to the input nodes connected to the output node and weights set in the link corresponding to each of the input nodes.

As described above, in the neural network 800, one or more nodes are interconnected through one or more links to form a relationship of an input node and an output node in the neural network. A characteristic of the neural network 800 may be determined according to the number of nodes and links in the neural network 800, a correlation between the nodes and the links, and a weight assigned to each of the links. For example, when there are two neural networks 800 each of which has the same number of nodes and the same number of links and has a different weight between the links, the two neural networks 800 may be recognized to be different from each other.

According to the exemplary embodiment of the present disclosure, the sleeping adequacy information generation module 151 may generate post-operation sleeping adequacy information of the user based on post-operation state information. In detail, the sleeping adequacy information generation module 151 may generate post-operation sleeping adequacy information based on post-operation state information generated based on the measurement information measured through the sensor unit 140 while the operation unit 120 performs the operation. In this case, the post-operation sleeping adequacy information may be generated in response to the post-operation state information measured during one cycle of the sleep cycle rhythm. Accordingly, the post-operation sleeping adequacy information may be formed of information about the sleeping state of the user during one cycle of the sleep cycle rhythm. For example, as illustrated in FIG. 5, the post-operation sleeping adequacy information may include information on each cycle of the sleep cycle rhythm. Further, the post-operation sleeping adequacy information may include information representing the ratio for each sleep stage of the user for each cycle as illustrated in Table 1 of FIG. 5.

According to the exemplary embodiment of the present disclosure, the sleeping adequacy information generation module 151 may generate reference sleeping adequacy information. In particular, the sleeping adequacy information generation module 151 may generate reference sleeping adequacy information that is a goal of the post-operation sleeping adequacy information. That is, the reference sleeping adequacy information may be information on the sleep cycle rhythm in which light sleep is minimum. For example, the reference sleeping adequacy information may mean the state in which deep sleep is 70% and REM sleep is 30%. Further, for example, the reference sleeping adequacy information may also mean the state where light sleep is 0%. The foregoing reference sleeping adequacy information is merely illustrative, and the present disclosure is not limited thereto. Further, the sleeping adequacy information generation module 151 may generate reference sleeping adequacy information based on predetermined information, and may also generate user-customized reference sleeping adequacy information according to a sleeping pattern of the user. For example, when the sleeping adequacy information generation module 151 generates reference sleeping adequacy information based on predetermined information, the user may select the predetermined reference sleeping adequacy information. In detail, the user may select variously classified reference sleeping adequacy information, such as age, gender, and location. For another example, when the sleeping adequacy information generation module 151 generates user-customized reference sleeping adequacy information, the sleeping adequacy information generation module 151 may generate reference sleeping adequacy information based on the biometric signal that maximizes sleep efficiency of the user. That is, the reference sleeping adequacy information may be the information that maximizes efficiency of the user's overall sleep, such as an increase in deep sleep, a significant decrease in awakening rate during sleep, and an increase in REM sleep. According to the exemplary embodiment of the present disclosure, the operation adequacy determination module 153 may determine adequacy of the operation by comparing the post-operation sleeping adequacy information with the reference sleeping adequacy information. In particular, the operation adequacy determination module 153 may determine similarity by comparing the post-operation sleeping adequacy information during one cycle of the sleep cycle rhythm with the reference sleeping adequacy information. In detail, the operation adequacy determination module 153 may determine similarity by comparing a ratio of each sleep stage of the post-operation sleeping adequacy information with a ratio of each sleep stage of the reference sleeping adequacy information. In this case, each sleep stage may include a light sleep stage, a normal sleep stage, a deep sleep stage, and an REM sleep stage. For example, the operation adequacy determination module 153 may determine similarity by comparing the ratios of light sleep, normal sleep, deep sleep, and REM sleep of the post-operation sleeping adequacy information including information during one cycle of the sleep cycle rhythm of the user after the environment control operation with the ratios of light sleep, normal sleep, deep sleep, and REM sleep of the reference sleeping adequacy information. The method of determining the similarity may be at least one of a time-series similarity determination method of measuring similarity according to a flow of time during one cycle, a method of determining similarity through a comparison of a ratio of each sleep stage during one cycle, and a graph similarity measurement method of determining similarity by calculating a distance of each corresponding point in a sleep cycle rhythm graph of the post-operation sleeping adequacy information and a sleep cycle rhythm graph of the reference sleeping adequacy information. That is, the operation adequacy determination module 153 may determine whether the sleeping adequacy information of the user according to the operation of the operation unit is close to the reference sleeping adequacy information (that is, the ideal sleeping state) and determine whether the operation determined by the operation information determination module improves the quality of sleep of the user (reward). The description of the method of determining the similarity is merely illustrative, and the present disclosure is not limited thereto.

According to the exemplary embodiment of the present disclosure, the operation adequacy determination module 153 may update the operation determination algorithm through the determination on adequacy for the operation. The operation adequacy determination module 153 may update the operation determination algorithm. In particular, when the similarity is high based on the determination on the similarity, the operation adequacy determination module 153 may update the operation determination algorithm so that a probability that the operation determination algorithm determines the corresponding operation information based on the current sleeping adequacy information increases. Further, when the similarity is low, the operation adequacy determination module 153 may update the operation determination algorithm so that a probability that the operation determination algorithm determines the operation information based on the current sleeping adequacy information decreases. The operation adequacy determination module 153 may update a weight of the artificial neural network of the operation determination algorithm and update the operation determination algorithm. For example, when the similarity obtained through the comparison between the post-operation sleeping adequacy information during one cycle of the sleep cycle rhythm and the reference sleeping adequacy information is 98%, the quality of sleep of the user is improved by the performance of the corresponding operation information, so that the operation adequacy determination module 153 may update the operation determination algorithm so that a probability that the corresponding operation information is selected increases. Further, when the similarity obtained through the comparison between the post-operation sleeping adequacy information during one cycle of the sleep cycle rhythm and the reference sleeping adequacy information is 14%, the operation adequacy determination module 153 may update the operation determination algorithm so that a probability that the corresponding operation information is selected decreases. The operation adequacy determination module 153 may increase the probability that the corresponding operation information is selected as the similarity is high, and decrease the probability that the corresponding operation information is selected as the similarity is low. For example, during the previous sleep cycle rhythm, when the user had a lot of tossing and turning time and relatively little deep sleep time, the operation information during the previous sleep cycle is the operation information degrading the quality of sleep of the user, so that the operation adequacy determination module 153 may update the operation determination algorithm so that the operation information during the previous sleep cycle is not selected. In this case, the operation information determination module may determine to perform an operation of relatively increasing a temperature for a predetermined portion of an initial period from a time point at which one cycle of the sleep cycle rhythm starts so that the tossing time of the user decreases according to the updated operation determination algorithm. When the tossing time of the user decreases according to the sensing result of the sensor unit after the operation of increasing the temperature for the predetermined portion of the initial period from the time point at which one cycle of the sleep cycle rhythm starts is performed, the operation adequacy determination module may determine that the corresponding operation is adequate, and update the operation determination algorithm so that a probability that the operation of increasing the temperature for the predetermined portion of the initial period is performed increases (that is, an operation similar to the previous operation which had drawn the preferable result in the similar environment is performed) in the case where the user tosses and turns a lot (that is, in the similar environment).

According to the exemplary embodiment of the present disclosure, the memory 130 may store a program code executable in the processor 150 and user data. For example, the memory 130 may store corresponding environment data from an environment related to the user and user data related to history information from the sleep of the user.

According to the exemplary embodiment of the present disclosure, the input unit 160 may be provided in the sleeping environment control device 100 using reinforcement learning, and may be formed of an input button, a touch screen, or a combination thereof. The input unit 160 may receive information about the user. For example, the input unit 160 may receive user information about at least one of a user's age, a user's gender, a user's region, and a survey result from the user. Herein, the survey result may be result information about a survey for recognizing the sleep cycle rhythm of the user. Further, the input unit 160 may receive at least one of information about a sleeping pattern, bedtime, and a wake-up time. The information input through the input unit 160 is merely illustrative, and the present disclosure is not limited thereto.

Further, the input unit 160 may be provided in the sleeping environment control device 100 using reinforcement learning in the form of an input button and a touch screen. A location at which the input unit 160 is provided may be one lateral surface of the sleeping environment control device 100 using reinforcement learning through which the user may easily input user information, and the present disclosure is not limited thereto.

According to the exemplary embodiment of the present disclosure, the network connection unit 170 may receive user information input from an external device or an application, not the sleeping environment control device 100 using reinforcement learning. Examples of the external device may be a Personal Computer (PC), a portable terminal, a wearable device, and the like. Further, the network connection unit 170 may establish a channel for data communication between the sensor unit 140, the operation unit 120, the processor 150, and the input unit 160.

According to the exemplary embodiment of the present disclosure, the network connection unit 170 may receive control information from at least one of the server 10 and the user terminal 20. More particularly, the network connection unit 170 may include a wired/wireless Internet module for a network connection. As the wireless Internet technology, wireless LAN (WLAN, Wi-Fi), a wireless broadband (Wibro), world interoperability for microwave access (Wimax), and high speed downlink packet access (HSDPA), and the like may be used. As the wired Internet technology, a digital subscriber line (XDSL), fibers to the home (FTTH), power line communication (PLC), and the like may be used.

Further, the network connection unit 170 may include a short-range communication module and transceive data with an electronic device which is located relatively close to the user terminal 20 and includes a short-range communication module. As a short range communication technology, a Bluetooth, radio frequency identification (RFID), infrared data association (IrDA), ultra wideband (UWB), ZigBee, and the like may be used.

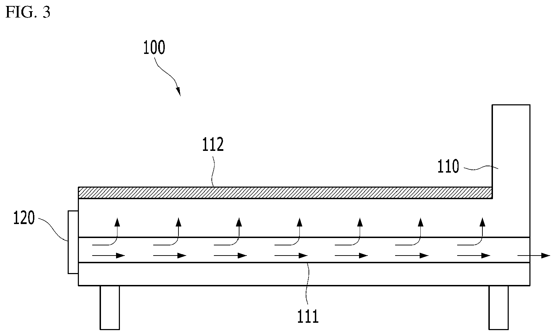

FIG. 3 is a diagram illustrating an example of the sleeping environment control device 100 using reinforcement learning according to the exemplary embodiment of the present disclosure.

According to the exemplary embodiment of the present disclosure, the sleeping environment control device 100 using reinforcement learning may control a temperature and humidity of the main body 110 in order to change the sleeping state of the user.

As illustrated in FIG. 3, the internal passage 111 may be provided inside the main body 110. The internal passage 111 may be a space in which hot wind or cold wind supplied from the operation unit 120 moves. The hot wind and cold wind provided form the operation unit 120 may pass through an upper end of the internal passage 111 to adjust a temperature of the main body 110. Further, the temperature of the hot wind and cold wind passing through the upper end of the internal passage 111 may be maintained by the bedclothes 112. That is, the sleeping environment control device 100 using reinforcement learning may bring a high-efficiency sleeping environment change with a minimal operation.

According to the exemplary embodiment of the present disclosure, the operation unit 120 may be provided in the main body 110. The location at which the operation unit 120 is provided may be separated from the main body or may be a lower portion of the main body 110. For example, as illustrated in FIG. 3, when the user lies on the main body 110, the location at which the operation unit 120 is provided may be the lower end of the main body where the user's feet are located. The location at which the operation unit 120 is provided is merely illustrative, and the present disclosure is not limited thereto.

Further, the operation unit 120 may adjust at least one of a temperature and humidity of the main body 110. For example, the operation unit 120 may supply at least one of hot wind and cold wind to the main body 110 supporting the user in order to control a body temperature of the user. Further, the operation unit 120 may perform at least one of a dehumidification operation and a humidification operation so as to control humidity of the main body 110 supporting the user. Accordingly, the operation unit 120 may control the body temperature of the user and the humidity to improve sleep efficiency.

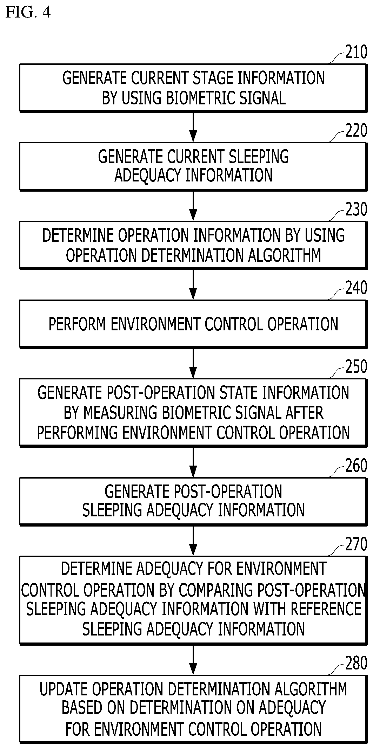

FIG. 4 is a flowchart illustrating a sleeping environment control method using reinforcement learning according to an exemplary embodiment of the present disclosure.

The sleeping environment control device 100 using reinforcement learning may measure and determine a sleeping state of a user and control a sleeping environment of the user in response to the determined sleeping state. The sleeping environment control device 100 using reinforcement learning may measure the sleeping state of the user. Further, the sleeping environment control device 100 using reinforcement learning may generate information on a sleeping state through the measured information of the user and determine a quality of the sleep. The sleeping environment control device 100 using reinforcement learning may provide the user with a sleeping environment that improves sleep efficiency through the determination on the quality of the sleep of the user.

According to the exemplary embodiment of the present disclosure, the sleeping environment control device 100 using reinforcement learning may measure a biometric signal of the user (210, 250). In particular, when the user sleeps, the sleeping environment control device 100 using reinforcement learning may measure at least one of a heart rate signal, a brainwave signal, a respiration signal, a movement signal of the user. Further, the sleeping environment control device 100 using reinforcement learning may measure a body temperature of the user and a temperature and humidity indoors in which the user sleeps. The sleeping environment control device 100 using reinforcement learning may generate current state information based on the measurement information.

According to the exemplary embodiment of the present disclosure, the sleeping environment control device 100 using reinforcement learning may generate current sleeping adequacy information (220). In detail, the sleeping environment control device 100 using reinforcement learning may generate current sleeping adequacy information based on the current state information generated based on the measurement information measured through the sensor unit 140. In this case, the current sleeping adequacy information may be generated in response to the current state information measured during one cycle of the sleep cycle rhythm. Accordingly, the current sleeping adequacy information may include information about a sleeping state of the user during one cycle of the sleep cycle rhythm. For example, the current sleeping adequacy information may be information including a ratio of a sleep stage of the user during one cycle.

According to the exemplary embodiment of the present disclosure, the sleeping environment control device 100 using reinforcement learning may determine operation information by using an operation determination algorithm (230). The operation determination algorithm is formed of an artificial neural network, and output a score of one or more pieces of candidate operation information by using the current sleeping adequacy information as an input. Further, the operation determination algorithm may determine operation information based on a score of each candidate operation information. In particular, the operation determination algorithm may output a score of corresponding various operation information based on the current sleeping adequacy information about the sleeping state of the user during one cycle of the sleep cycle rhythm, and determine operation information in which the score is maximum among the operation information. For example, when the scores for the operation information output by the operation determination algorithm based on the current sleeping adequacy information are 10, 8, 18, 2, 3, and 6, the operation determination algorithm may determine operation information by selecting 18 of the highest score among the operation information. The description of the numerical value of the score for the operation information is merely illustrative, and the present disclosure is not limited thereto. That is, the operation determination algorithm may have a plurality of operation options according to the current state of the user, and select the most adequate operation among the plurality of operation options as operation information by using the computation of the artificial neural network.

According to the exemplary embodiment of the present disclosure, the sleeping environment control device 100 using reinforcement learning may perform an environment control operation (240). The sleeping environment control device 100 using reinforcement learning may perform an environment control operation through the operation unit 120. The operation unit 120 may be provided in the main body 110 and perform at least one of a hot air supply operation, a cold air supply operation, a dehumidification operation, and a humidification operation in order to control a body temperature of the user and humidity. The operation unit 120 may perform the operation based on the operation information determined by the operation determination algorithm. For example, when the operation information determined by the operation determination algorithm is information that increases the body temperature of the user, the operation unit 120 may increase the body temperature of the user by supplying hot wind. For another example, when the operation information determined by the operation determination algorithm is information that lowers the body temperature of the user and increases humidity of a space in which the user is located, the operation unit 120 may lower the body temperature of the user and increase humidity of a space in which the user is located by supplying cold wind and performing the dehumidification operation.

According to the exemplary embodiment of the present disclosure, the sleeping environment control device 100 using reinforcement learning may generate post-operation sleeping adequacy information (250). In detail, the sleeping environment control device 100 using reinforcement learning may generate post-operation sleeping adequacy information based on post-operation state information generated based on the measurement information measured through the sensor unit 140 while the operation unit 120 performs the operation. In this case, the post-operation sleeping adequacy information may be generated in response to the post-operation state information measured during one cycle of the sleep cycle rhythm, and may also be generated in the unit of one day. Accordingly, the post-operation sleeping adequacy information may include information about the sleeping state of the user during one cycle of the sleep cycle rhythm. For example, the post-operation sleeping adequacy information may be information including a ratio of a sleep stage of each user during one cycle.

According to the exemplary embodiment of the present disclosure, the sleeping environment control device 100 using reinforcement learning may generate reference sleeping adequacy information. In particular, the sleeping environment control device 100 using reinforcement learning may generate reference sleeping adequacy information that is a goal of the post-operation sleeping adequacy information. In detail, the sleeping environment control device 100 using reinforcement learning may generate reference sleeping adequacy information based on a biometric signal that maximizes sleep efficiency of the user during one cycle of the sleep cycle rhythm and user input information. That is, the reference sleeping adequacy information may be the information that maximizes efficiency of the user's overall sleep, such as an increase in deep sleep, a significant decrease in awakening rate during sleep, and an increase in REM sleep.

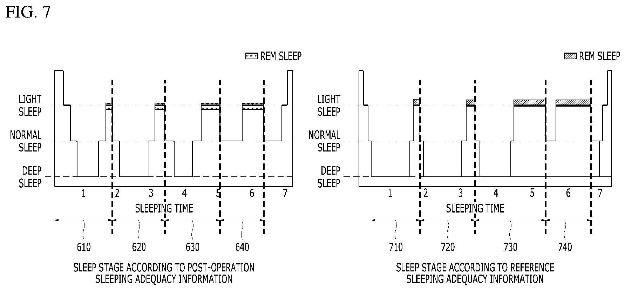

According to the exemplary embodiment of the present disclosure, the sleeping environment control device 100 using reinforcement learning may determine adequacy for the environment control operation by comparing the post-operation sleeping adequacy information with the reference sleeping adequacy information (260). The sleeping environment control device 100 using reinforcement learning may determine adequacy of the environment control operation through the operation adequacy determination module 153. In particular, the operation adequacy determination module 153 may determine similarity by comparing the post-operation sleeping adequacy information during one cycle of the sleep cycle rhythm with the reference sleeping adequacy information. In detail, the operation adequacy determination module 153 may determine similarity by comparing a ratio of each sleep stage of the post-operation sleeping adequacy information with a ratio of each sleep stage of the reference sleeping adequacy information. In this case, each sleep stage may include a light sleep stage, a normal sleep stage, a deep sleep stage, and an REM sleep stage. For example, as illustrated in FIG. 7, the sleep cycle rhythm of the post-operation sleeping adequacy information generated by measuring the sleeping state of the user may be represented by reference numerals 610, 620, 630, and 640. Further, the sleep cycle rhythm of the reference sleeping adequacy information that maximizes sleep efficiency of the user and is a goal of the post-operation sleeping adequacy information may be represented by reference numerals 710, 720, 730, and 740.

According to the exemplary embodiment of the present disclosure, as illustrated in FIG. 7, light sleep, normal sleep, deep sleep, and REM sleep according to the post-operation sleeping adequacy information and light sleep, normal sleep, deep sleep, and REM sleep according to the reference sleeping adequacy information may be compared in the form of graph. For example, in comparison between reference numeral 610 and reference numeral 710, it can be seen that the ratios of light sleep and normal sleep are relatively small, and the ratios of deep sleep and REM sleep are large. Further, it can be seen that as well as the comparison between the reference numeral 610 and reference numeral 710, the ratio aspects are similar in the comparisons between the reference numeral 620 and reference numeral 720, between the reference numeral 630 and reference numeral 730, and between the reference numeral 640 and reference numeral 740. That is, the reference sleeping adequacy information may be the information that is the goal of the post-operation sleeping adequacy information and about the sleep stage that maximizes sleep efficiency of the user.

According to the exemplary embodiment of the present disclosure, the sleeping environment control device 100 using reinforcement learning may determine adequacy of the operation by comparing the post-operation sleeping adequacy information with the reference sleeping adequacy information. In particular, the sleeping environment control device 100 using reinforcement learning may determine similarity by comparing the post-operation sleeping adequacy information during one cycle of the sleep cycle rhythm with the reference sleeping adequacy information. In detail, the sleeping environment control device 100 using reinforcement learning may compare a ratio of each sleep stage of a first cycle 610 of the post-operation sleeping adequacy information with a ratio of each sleep stage of a first cycle 710 of the reference sleeping adequacy information. For example, the sleeping environment control device 100 using reinforcement learning may determine similarity by comparing a ratio of each of light sleep, normal sleep, deep sleep, and REM sleep in reference numeral 610 with ratio of each of light sleep, normal sleep, deep sleep, and REM sleep in reference numeral 710. The method of determining the similarity may be, in particular, at least one of a time-series similarity determination method of measuring similarity according to a flow of time during one cycle, a method of determining similarity through a comparison of a ratio of each sleep stage during one cycle, and a graph similarity measurement method of determining similarity by calculating a distance of each corresponding point in a sleep cycle rhythm graph of the post-operation sleeping adequacy information and a sleep cycle rhythm graph of the reference sleeping adequacy information. The description of the method of determining the similarity is merely illustrative, and the present disclosure is not limited thereto.

Accordingly, the sleeping environment control device 100 using reinforcement learning may determine adequacy of the sleep of the user by comparing the post-operation sleeping adequacy information with the reference sleeping adequacy information through the foregoing method. For example, as the similarity between the post-operation sleeping adequacy information and the reference sleeping adequacy information is high, the current sleeping state is close to an ideal sleeping state, so that the sleeping environment control device 100 using reinforcement learning may determine that the environment control operation of the sleeping environment control device 100 using reinforcement learning is appropriate. The post-operation sleeping adequacy information and the reference sleeping adequacy information are merely illustrative, and the present disclosure is not limited thereto.

According to the exemplary embodiment of the present disclosure, the sleeping environment control device 100 using reinforcement learning may update the operation determination algorithm based on the determination on adequacy for the environment control operation (270). The sleeping environment control device 100 using reinforcement learning may update the operation determination algorithm through the operation adequacy determination module 153. In particular, when the similarity is high based on the determination on the similarity, the sleeping environment control device 100 using reinforcement learning may update the operation determination algorithm so that a probability that the operation determination algorithm determines the operation information based on the current sleeping adequacy information increases. Further, when the similarity is low, the sleeping environment control device 100 using reinforcement learning may update the operation determination algorithm so that a probability that the operation determination algorithm determines the operation information based on the current sleeping adequacy information decreases. For example, when the similarity obtained through the comparison between the post-operation sleeping adequacy information during one cycle of the sleep cycle rhythm and the reference sleeping adequacy information is 98%, the sleeping environment control device 100 using reinforcement learning may update the operation determination algorithm so that a probability that the corresponding operation information is selected increases. Further, when the similarity obtained through the comparison between the post-operation sleeping adequacy information during one cycle of the sleep cycle rhythm and the reference sleeping adequacy information is 14%, the sleeping environment control device 100 using reinforcement learning may update the operation determination algorithm so that a probability that the corresponding operation information is selected decreases. The description of the numerical value of the similarity is merely illustrative, and the present disclosure is not limited thereto.

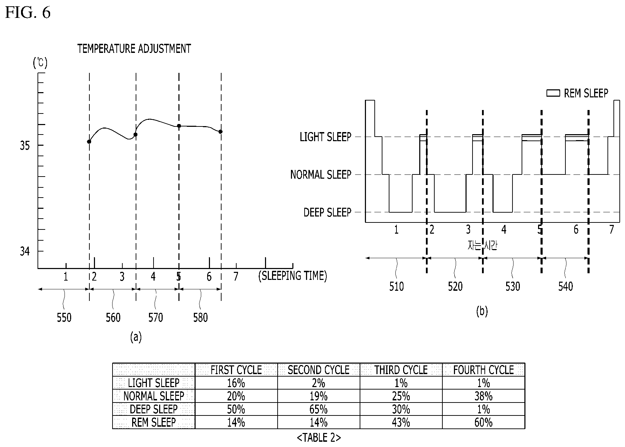

FIG. 6 is a diagram illustrating an example of a sleep stage change of a user according to a temperature control change of the sleeping environment control device 100 using reinforcement learning according to the exemplary embodiment of the present disclosure.

According to the exemplary embodiment of the present disclosure, the sleeping environment control device 100 using reinforcement learning may change a skin temperature of the user. In particular, the sleeping environment control device 100 using reinforcement learning may finely change a skin temperature of the user by performing the sleeping environment change operation. For example, the sleeping environment control device 100 using reinforcement learning may change a skin temperature of the user by supplying at least one of hot wind and cold wind to the user. When the skin temperature of the user finely increases by 0.4°C, the sleeping environment control device 100 using reinforcement learning may help to lower the body temperature of the user. Normally, the body temperature decreases during nighttime sleep due to circadian rhythm, and the increase in the skin temperature accelerates the decrease in the body temperature of the user, so that sleep efficiency is increased. Accordingly, the sleeping environment control device 100 using reinforcement learning may facilitate the decrease in the body temperature by increasing the skin temperature of the user, thereby improving sleep efficiency of the user.

According to the exemplary embodiment of the present disclosure, as illustrated in (a) of FIG. 6, the sleeping environment control device 100 using reinforcement learning may increase a skin temperature of the user during a predetermined cycle. Further, as illustrated in FIG. 6, the sleep stage ratio of the user may be changed in response to the increased temperature change.

FIG. 6 is a diagram illustrating an example of a sleep stage change of a user according to a temperature control change of the sleeping environment control device 100 using reinforcement learning according to the exemplary embodiment of the present disclosure.
(a) of FIG. 6 is a graph representing that the sleeping environment control device 100 using reinforcement learning controls a temperature by performing the temperature adjusting operation according to time. Further, (b) of FIG. 6 may be a graph for sleeping adequacy information obtainable through the sensing when the sleeping environment control device 100 using reinforcement learning performs the temperature adjustment like (a) of FIG. 6. Table 2 illustrated in FIG. 6 may be the ratios drawn based on the sleep ratios represented for each cycle in (b) of FIG. 6.

According to the exemplary embodiment of the present disclosure, as illustrated by reference numeral 550 of FIG. 6, the sleeping environment control device 100 using reinforcement learning may not perform the temperature adjusting operation. In particular, the sleeping environment control device 100 using reinforcement learning may obtain sleeping adequacy information of the user during the first cycle of the sleep cycle rhythm through the sensing. The sleeping adequacy information of the user for the first cycle obtained by the sleeping environment control device 100 using reinforcement learning may be the same as the region denoted by reference numeral 510, and the sleep stage ratio in the corresponding region may be the same as a first cycle of Table 2 of FIG. 6. After the sleeping adequacy information of the user during the first cycle of the sleep cycle rhythm is obtained, the sleeping environment control device 100 using reinforcement learning may determine an environment control operation to be performed through the operation determination algorithm based on the sleeping adequacy information 510 of the first cycle. The sleeping environment control device 100 using reinforcement learning may perform the sleeping environment control operation during the second cycle through the determined operation. As illustrated in (a) of FIG. 6, the sleeping environment control device 100 using reinforcement learning may perform the sleeping environment control operation in the second cycle like reference numeral 560. The second-cycle sleeping adequacy information 520 of the user is illustrated in (b) of FIG. 6, and as illustrated in Table 2 of FIG. 6, the ratio of deep sleep of the user may increase compared to the first cycle. Accordingly, the sleeping environment control device 100 using reinforcement learning may determine that the corresponding sleeping environment control operation is adequate. The sleeping environment control device 100 using reinforcement learning may adjust a weight of the artificial neural network 800 so that a similar operation is selected in a similar situation based on the result of the determination that the environment control operation for the second cycle in the sleep is adequate. Accordingly, as illustrated in (a) of FIG. 6, the sleeping environment control device 100 using reinforcement learning may perform the sleeping environment control operation similar to that of the second cycle in the third cycle like reference numeral 570. Accordingly, sleeping adequacy information of the third cycle may be the same as reference numeral 530 in (b) of FIG. 6. Further, the sleep stage ratio of the user may represent that in the third cycle, the ratio of deep sleep of the user decreases, but the ratio of REM sleep further increases as represented in Table 2 of FIG. 6. The ratio of REM sleep dramatically increases in the third cycle and the ratio of deep sleep decreases in the third cycle, so that the sleeping environment control device 100 using reinforcement learning may determine that the decrease in the ratio of deep sleep is inadequate in the corresponding sleeping environment control operation, and the increase in the ratio of the REM sleep is adequate. The sleeping environment control device 100 using reinforcement learning may determine that there is a probability that the higher maximum temperature in the temperature control operation 570 during the third cycle than the maximum temperature in the temperature control operation 560 during the second cycle is related to the decrease in the deep sleep in time, and determine the maximum temperature of the temperature control operation 570 during the fourth cycle to be similar to the maximum temperature of the temperature control operation 560 during the second cycle. That is, a final goal of the sleeping environment control device 100 using reinforcement learning of the present disclosure is to increase both the ratio of the deep sleep and the ratio of the REM sleep, so that the sleeping environment control device 100 using reinforcement learning may update the operation determination algorithm of the environment control operation so as to increase both the ratio of the deep sleep and the ratio of the REM sleep. Accordingly, the sleeping adequacy information of the fourth cycle may be the same as reference numeral 540. As represented in Table 2 of FIG. 6, the deep sleep and the REM sleep during the fourth cycle may decrease compared to the third cycle. Accordingly, the sleeping environment control device 100 using reinforcement learning may determine that the corresponding operation is inadequate. The sleeping environment control device 100 using reinforcement learning may adjust the weight of the artificial neural network 800 so that the similar operation is not selected in the similar situation based on the result of the determination that the corresponding operation is inadequate.

That is, the sleeping environment control device 100 using reinforcement learning may control the sleeping environment of the user based on the repetition of the reinforcement learning. In this case, the increase in the quantity of deep sleep and REM sleep in the sleep cycle rhythm may be the goal of the reinforcement learning. Further, the increase in the quantity of at least one of the deep sleep and the REM sleep by the sleeping environment control operation of the sleeping environment control device 100 using reinforcement learning may be a reward. Accordingly, the sleeping environment control device 100 using reinforcement learning may provide the user with the optimum sleeping environment through the repeated reinforcement learning. The sleep ratio of the user is merely illustrative, and the present disclosure is not limited thereto.

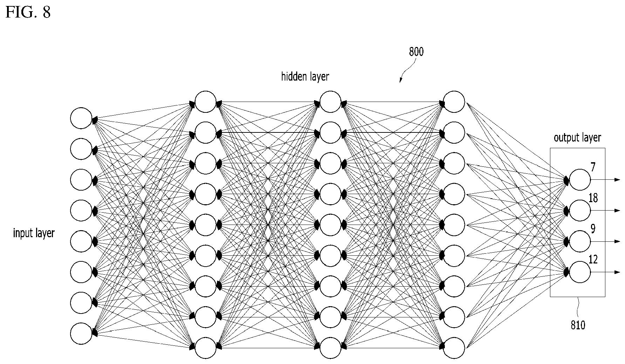

FIG. 8 is a configuration diagram illustrating the artificial neural network 800 configuring the operation determination algorithm according to the exemplary embodiment of the present disclosure.

The neural network 800 consists of one or more nodes. The nodes (or neurons) configuring the neural networks may be interconnected by one or more "links".

In the neural network 800, one or more nodes connected through the links may relatively form a relationship with an input node and an output node. The concept of the input node is relative to the concept of the output node, and a predetermined node having an output node relationship with respect to one node may have an input node relationship in a relationship with another node, and a reverse relationship is also available. As described above, the relationship between the input node and the output node may be generated based on the link. One or more output nodes may be connected to one input node through a link, and a reverse case may also be valid.

In the relationship between an input node and an output node connected through one link, a value of the output node may be determined based on data input to the input node. Herein, a node connecting the input node and the output node may have a weight. The weight is variable, and in order for the neural network 800 to perform a desired function, the weight may be varied by a user or an algorithm. For example, when one or more input nodes are connected to one output node by links, respectively, a value of the output node may be determined based on values input to the input nodes connected to the output node and weights set in the link corresponding to each of the input nodes.

As described above, in the neural network 800, one or more nodes are connected with each other through one or more links to form a relationship with an input node and an output node in the neural network 800. A characteristic of the neural network 800 may be determined according to the number of nodes and links in the neural network 800, a correlation between the nodes and the links, and a weight assigned to each of the links. For example, when there are two neural networks 800 each of which has the same number of nodes and the same number of links and has a different weight between the links, the two neural networks 800 may be recognized to be different from each other.

As illustrated in FIG. 8, the neural network 800 may be formed of one or more nodes. Some of the nodes configuring the neural network 800 may form one layer based on distances from an initial input node. For example, a set of nodes having a distance of n from an initial input node may form n layers. The distance from the initial input node may be defined by the minimum number of links, which needs to be passed from the initial input node to a corresponding node. However, the definition of the layer is arbitrary for the description, and a degree of the layer in the neural network 800 may be defined by a different method from the foregoing method. For example, the layers of the nodes may be defined by a distance from a final output node.

The initial input node may mean one or more nodes to which data is directly input without passing through a link in a relationship with other nodes among the nodes in the neural network 800. Otherwise, the initial input node may mean nodes which do not have other input nodes connected through the links in a relationship between the nodes based on the link in the neural network 800. Similarly, the final output node may mean one or more nodes which do not have an output node in a relationship with other nodes among the nodes in the neural network 800. Further, the hidden node may mean nodes configuring the neural network 800, not the initial input node and the final output node.

The neural network 800 according to the exemplary embodiment of the present disclosure may have the neural network in the form in which the number of nodes of the input layer may be larger than the number of nodes of the hidden layer close to the output layer, and the number of nodes decreases from the input layer to the hidden layer.

In the exemplary embodiment of the present disclosure, the neural network 800 may include MultiLayer Perceptron (MLP), a Recurrent Neural Network (RNN), a Convolutional Neural Network (CNN), and the like so that data processing is possible. Further, in the exemplary embodiment of the present disclosure, in order to prevent performance deterioration due to a long range dependency vanishing gradient incurable according to an increase in a length of an event, the neural network 800 of the present disclosure may include a Long Short-Term memory (LSTM). Further, in the exemplary embodiment of the present disclosure, as the optimizing method of the artificial neural network method, Stochastic Gradient Descent (SGD), Momentum, Adam, AdaGrad, RMSprop, and the like may be used. Further, training data D may be learned only once, and parameters minimizing an error function may also be obtained through several- time repeated epoch, and whether to end the learning stage may be determined after whether the sufficient optimization has been made is inspected.

In the RNN, unlike the general feed-forward neural network, an output layer of the hidden layer may be an input of the same hidden layer again. The RNN is the neural network which performs a computation in consideration of current input input data and previously input data at the same time, and has a feedback structure and has a memory ability. Accordingly, the RNN may be trained so as to interpret current data according to a meaning of previous data in the data. The LSTM that is one of the RNNs is also called a long short term memory network, and learn long-term dependency. In the exemplary embodiment of the present disclosure, the neural network may include a predetermined neural network, such as a depth gated RNN and a clockwork RNN, capable of processing data, as well as the LSTM that is one of the RNNs.

The neural network 800 of FIG. 8 may include one or more hidden layers. A hidden node of the hidden layer may use an output of a previous layer and an output of a surrounding hidden layer as an input. In the respective hidden layers, the numbers of hidden nodes may also be the same as or different from each other. The number of nodes of the input layer may be determined based on the number of data fields of the input data, and may be the same as or different from the number of hidden nodes. The input data input to the input layer may be computed by the hidden node of the hidden layer, and may be output by a Fully Connected Layer (FCL) that is the output layer.

According to the exemplary embodiment of the present disclosure, the operation determination algorithm may output a score of each of one or more pieces of candidate operation information by using the current sleeping adequacy information as an input. Further, the operation determination algorithm may determine operation information based on the score of each candidate operation information. In particular, the operation determination algorithm may output a score of corresponding various operation information based on the current sleeping adequacy information about the sleeping state of the user during one cycle of the sleep cycle rhythm, and determine operation information in which the score is maximum among the operation information. For example, as illustrated in FIG. 8, when the number of operation information output by the operation determination algorithm based on the current sleeping adequacy information is four, and the scores for the output operations are 7, 18, 9, and 12, the operation determination algorithm may select 18 of the highest score in the operation information and determine operation information. The description of the numerical value of the score for the operation information is merely illustrative, and the present disclosure is not limited thereto. That is, the operation determination algorithm may have a plurality of operation options according to the current state of the user, and select the most adequate operation among the plurality of operation options as operation information by using the computation of the artificial neural network.

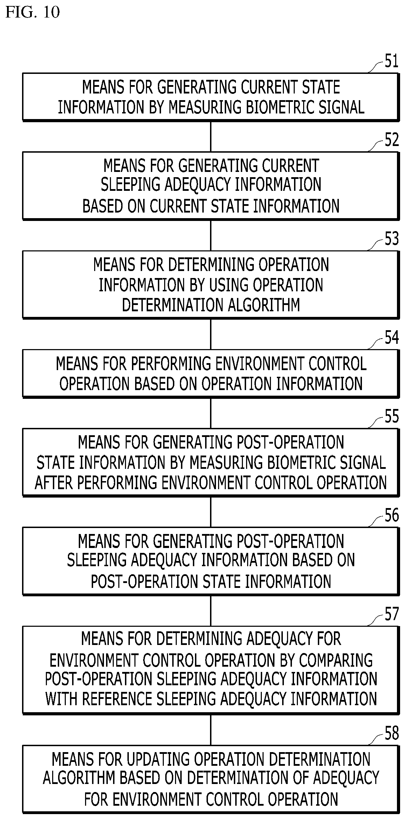

FIG. 10 is a block diagram illustrating a means for implementing the sleeping environment control method using reinforcement learning according to the exemplary embodiment of the present disclosure.

According to the exemplary embodiment of the present disclosure, the sleeping environment control device 100 using reinforcement learning may be implemented by a means 51 for generating current state information and post-operation state information by measuring a biometric signal; a means 52 for generating current sleeping adequacy information based on the current state information; a means 53 for determining operation information by using an operation determination algorithm; a means 54 for performing an environment control operation based on the operation information; a means 55 for generating post-operation state information by measuring a biometric signal after performing the environment control operation; a means 56 for generating post-operation sleeping adequacy information based on the post-operation state information; a means 57 for determining adequacy for the environment control operation by comparing the post-operation sleeping adequacy information with reference sleeping adequacy information; and a means 58 for updating the operation determination algorithm based on the determination of the adequacy for the environment control operation.

Alternatively, the means for generating the current state information and the post-operation state information may include at least one of a user state measurement sensor which measures at least one of a heart rate, a respiration rate, movement, and brain waves of the user in a contact or non-contact manner, a temperature sensor which measures at least one of a temperature indoors in which the user sleeps and a body temperature of the user, and a humidity sensor that measures humidity indoors in which the user sleeps, and at least one of the current state information, indoor temperature information, and indoor humidity information may be obtained through at least one sensor among the sensors.

Alternatively, the means for performing the environment control operation based on the operation information may be provided in the main body, and may supply at least one of hot wind and cold wind to the main body in order to control the body temperature of the user.

Alternatively, the means for generating the current sleeping adequacy information based on the current state information may include at least one of the respiration state information, the heart rate state information, the brain wave information, and the movement state information of the user measured in the means for generating the current state information and the post-operation state information, and include a measurement result during one cycle of a sleep cycle rhythm of the user.

Alternatively, the means for generating the current sleeping adequacy information based on the current state information may be generated based on the current state information measured during one cycle of a sleep cycle rhythm.

Alternatively, the means for generating the post-operation state information by measuring the biometric signal after the environment control operation is performed may generate the post-operation state information based on the biometric signal during one cycle of the sleep cycle rhythm in which the means for performing the environment control operation performs an operation by a control signal of the processor 150.

Alternatively, the means for generating the post-operation state information by measuring the biometric signal after the environment control operation is performed may be generated based on the post-operation state information measured during one cycle of the sleep cycle rhythm.

Alternatively, the reference sleeping adequacy information may be generated based on a biometric signal that maximizes sleep efficiency of the user during one cycle of the sleep cycle rhythm, and may be a goal of the post-operation sleeping adequacy information.

Alternatively, the operation determination algorithm may be formed of an artificial neural network, and may output a score of each of one or more pieces of candidate operation information by using the current sleeping adequacy information as an input, and determine the operation information based on the score of each of the candidate operation information.

Alternatively, the means for determining adequacy for the environment control operation may determine adequacy for the operation by comparing the post-operation sleeping adequacy information during one cycle of the sleep cycle rhythm with the reference sleeping adequacy information and determining similarity.

Alternatively, the means for updating the operation determination algorithm based on the determination on the adequacy for the environment control operation may update the operation determination algorithm so that a probability that the operation determination algorithm determines the operation information based on the current sleeping adequacy information increases when the similarity is high, or update the operation determination algorithm so that a probability that the operation determination algorithm determines the operation information based on the current sleeping adequacy information decreases when the similarity is low.

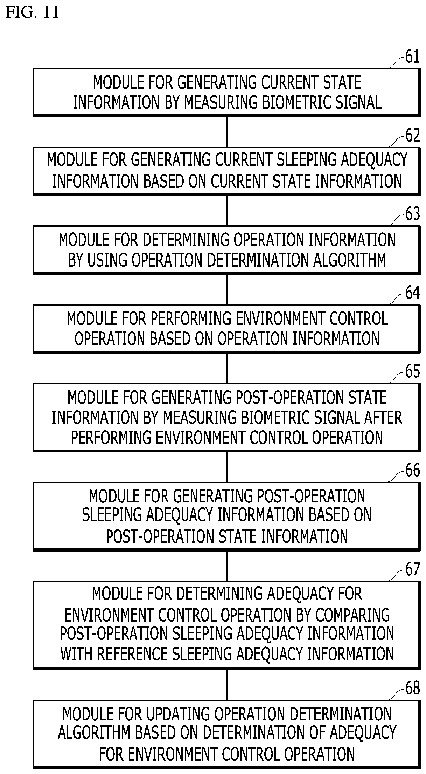

FIG. 11 is a block diagram illustrating a module for implementing the sleeping environment control method using reinforcement learning according to the exemplary embodiment of the present disclosure.

The sleeping environment control method using reinforcement learning according to the exemplary embodiment of the present disclosure may be implemented by following modules.

According to an exemplary embodiment of the present disclosure, the sleeping environment control device 100 using reinforcement learning may be implemented by a module 61 for generating current state information and post-operation state information by measuring a biometric signal; a module 62 for generating current sleeping adequacy information based on the current state information; a module 63 for determining operation information by using an operation determination algorithm; a module 64 for performing an environment control operation based on the operation information; a module 65 for generating post-operation state information by measuring a biometric signal after performing the environment control operation; a module 66 for generating post-operation sleeping adequacy information based on the post-operation state information; a module 67 for determining adequacy for the environment control operation by comparing the post-operation sleeping adequacy information with reference sleeping adequacy information; and a module 68 for updating the operation determination algorithm based on the determination of the adequacy for the environment control operation.

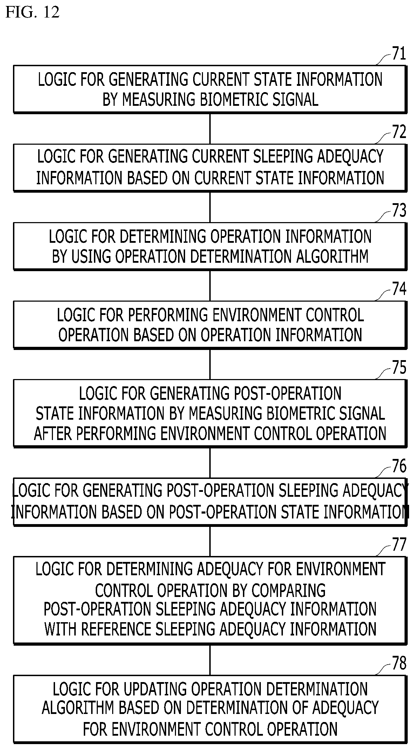

FIG. 12 is a block diagram illustrating logic for implementing the sleeping environment control method using reinforcement learning according to the exemplary embodiment of the present disclosure.

The sleeping environment control method using reinforcement learning according to the exemplary embodiment of the present disclosure may be implemented by following logic.

According to an exemplary embodiment of the present disclosure, the sleeping environment control device 100 using reinforcement learning may be implemented by logic 71 for generating current state information and post-operation state information by measuring a biometric signal; logic 72 for generating current sleeping adequacy information based on the current state information; logic 73 for determining operation information by using an operation determination algorithm; logic 74 for performing an environment control operation based on the operation information; logic 75 for generating post-operation state information by measuring a biometric signal after performing the environment control operation; logic 76 for generating post-operation sleeping adequacy information based on the post-operation state information; logic 77 for determining adequacy for the environment control operation by comparing the post-operation sleeping adequacy information with reference sleeping adequacy information; and logic 78 for updating the operation determination algorithm based on the determination of the adequacy for the environment control operation.

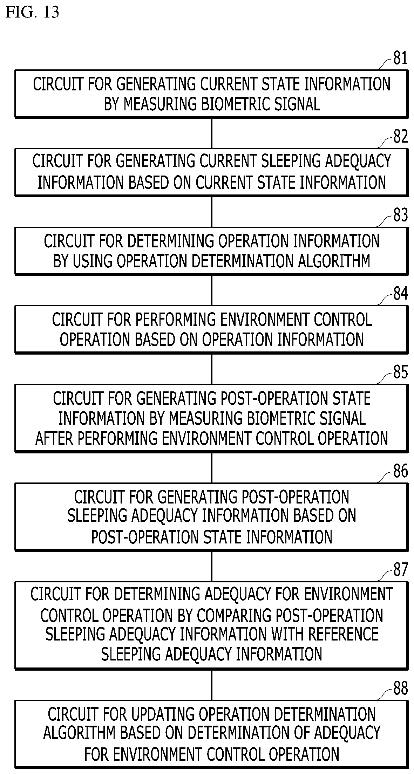

FIG. 13 is a block diagram illustrating a circuit for implementing the sleeping environment control method using reinforcement learning according to the exemplary embodiment of the present disclosure.

The sleeping environment control method using reinforcement learning according to the exemplary embodiment of the present disclosure may be implemented by following circuits.

According to an exemplary embodiment of the present disclosure, the sleeping environment control device 100 using reinforcement learning may be implemented by a circuit 81 for generating current state information and post-operation state information by measuring a biometric signal; a circuit 82 for generating current sleeping adequacy information based on the current state information; a circuit 83 for determining operation information by using an operation determination algorithm; a circuit 84 for performing an environment control operation based on the operation information; a circuit 85 for generating post-operation state information by measuring a biometric signal after performing the environment control operation; a circuit 86 for generating post-operation sleeping adequacy information based on the post-operation state information; a circuit 87 for determining adequacy for the environment control operation by comparing the post-operation sleeping adequacy information with reference sleeping adequacy information; and a circuit 88 for updating the operation determination algorithm based on the determination of the adequacy for the environment control operation.

Those skilled in the art shall recognize that the various illustrative logical blocks, configurations, modules, circuits, means, logic, and algorithm operations described in relation to the exemplary embodiments additionally disclosed herein may be implemented by electronic hardware, computer software, or in a combination of electronic hardware and computer software. In order to clearly exemplify interchangeability of hardware and software, the various illustrative components, blocks, configurations, means, logic, modules, circuits, and operations have been generally described above in the functional aspects thereof. Whether the functionality is implemented as hardware or software depends on a specific application or design restraints given to the general system. Those skilled in the art may implement the described functionality in various methods for each specific application, but such implementation decisions should not be interpreted as departing from the scope of the present disclosure.

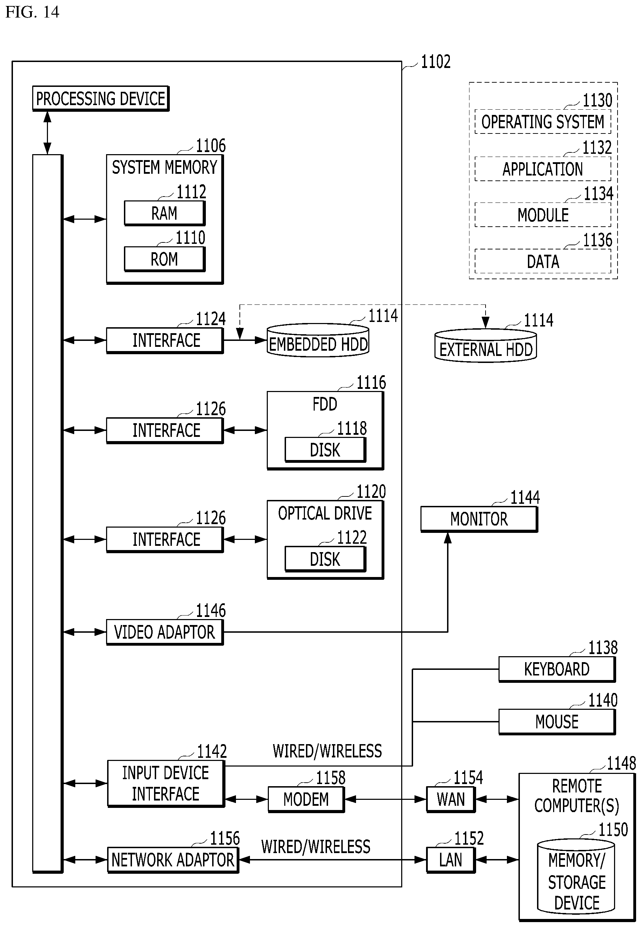

FIG. 14 is a simple and general schematic diagram illustrating an example of a computing environment in which the exemplary embodiments of the present disclosure are implementable.

The present invention has been described in relation to the computer executable command generally executable in one or more computers, but those skilled in the art will be well appreciated that the present invention may be combined with other program modules and/or implemented by a combination of hardware and software.

In general, a module in the present specification includes a routine, a procedure, a program, a component, a data structure, and the like performing a specific task or implementing a specific abstract data form. Further, those skilled in the art will appreciate well that the method of the present invention may be carried out by a single-processor or multiprocessor computer system, a minicomputer, and a main frame computer, and other computer system configurations, such as a personal computer, a hand-held computing device, microprocessor-based or programmable home appliances, and the like (each of which may be connected with one or more associated devices and be operated).

The exemplary embodiments of the present invention may be carried out in a distribution computing environment, in which specific tasks are performed by remote processing devices connected through a communication network. In the distribution computing environment, a program module may be positioned in both a local memory storage device and a remote memory storage device.

The computer generally includes various computer readable media. A computer accessible medium may be a computer readable medium regardless of the kind of medium, and the computer readable medium includes volatile and non-volatile media, transitory and non-transitory media, portable and non-portable media. As a non-limited example, the computer readable medium may include a computer readable storage medium and a computer readable transmission medium.

The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a Random Access Memory (RAM), a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium implements a computer readable command, a data structure, a program module, or other data in a modulated data signal, such as a carrier wave or other transport mechanisms, and generally includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, radio frequency (RF), infrared rays, and other wireless media. A combination of the predetermined media among the foregoing media is also included in a range of the computer readable transport medium.

An illustrative environment 1100 including a computer 1102 and implementing various aspects of the present invention is represented, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various common processors. A dual processor and other multi-processor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an erasable and programmable ROM (EPROM), and an EEPROM, and the BIOS includes a basic routine helping a transport of information among the constituent elements within the computer 1102 at a specific time, such as starting. The RAM 1112 may also include a high-rate RAM, such as a static RAM, for caching data.

The computer 1102 also include an embedded hard disk drive (HDD) 1114 (for example, enhanced integrated drive electronics (EIDE) and serial advanced technology attachment (SATA)) - the embedded HDD 1114 being configured for outer mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable storage media, the HDD, the portable magnetic disk, and the portable optical media, such as a CD, or a DVD, are mentioned, but those skilled in the art will well appreciate that other types of compute readable storage media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. Those skilled in the art will appreciate well that the present invention may be implemented by several commercially available operating systems or a combination of the operating systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a server computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as the Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication server on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.1 la) or 54 Mbps (802.11b) in an unauthorized 2.4 and 5 GHz wireless band, or may be operated in a product including both bands (dual bands).

Those skilled in the art will appreciate that the various illustrative logical blocks, modules, processors, means, circuits, and algorithm operations described in relation to the exemplary embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various exemplary embodiments presented herein may be implemented by a method, a device, or a manufactured article using a standard programming and/or engineering technology. A term "manufactured article" includes a computer program, a carrier, or a medium accessible from a predetermined computer-readable device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. A term "machine-readable medium" includes a wireless channel and various other media, which are capable of storing, holding, and/or transporting a command(s) and/or data, but is not limited thereto.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented exemplary embodiments is provided so as for those skilled in the art to use or carry out the present disclosure. Various modifications of the exemplary embodiments may be apparent to those skilled in the art, and general principles defined herein may be applied to other exemplary embodiments without departing from the scope of the present disclosure. Accordingly, the present disclosure is not limited to the exemplary embodiments suggested herein, and shall be interpreted within the broadest meaning range consistent to the principles and new characteristics presented herein.

### [Mode for Carrying Out the Invention]

The related contents have been described in the best mode for carrying out the invention as described above.

### [Industrial Availability]

The present disclosure relates to a device for controlling a sleeping environment so as to improve sleep efficiency of a user.

## Claims

1. A sleeping environment control device using reinforcement learning, comprising:
a main body on which a user is located;
a sensor unit configured to measure a biometric signal of the user and generate current state information and post-operation state information;
an operation unit configured to control a sleeping environment of the main body in order to change a sleeping state of the user based on a control signal of a processor;
a processor including one or more cores; and
a memory configured to store program codes executable in the processor,
wherein the processor includes:
a sleeping adequacy information generation module which generates current sleeping adequacy information of the user based on the current state information and generates post-operation sleeping adequacy information of the user based on the post-operation state information;
an operation information determination module which determines operation information controlling an operation of the operation unit by using an operation determination algorithm based on the current sleeping adequacy information; and
an operation adequacy determination module which updates the operation determination algorithm by comparing the post-operation sleeping adequacy information with reference sleeping adequacy information and determining adequacy for the operation.

2. The sleeping environment control device of claim 1, wherein the sensor unit includes at least one of a user state measurement sensor which measures at least one of a heart rate, a respiration rate, movement, and brain waves of the user in a contact or noncontact manner, a temperature sensor which measures at least one of a temperature indoors in which the user sleeps and a body temperature of the user, and a humidity sensor which measures humidity indoors in which the user sleeps, and
at least one of the current state information, indoor temperature information, and indoor humidity information is obtained through at least one of the sensors.

3. The sleeping environment control device of claim 1, wherein the operation unit is provided in the main body and supplies at least one of hot wind and cold wind to the main body in order to control a body temperature of the user.

4. The sleeping environment control device of claim 1, wherein the current state information includes at least one of respiration state information, heart rate state information, brain wave information, and movement state information of the user measured in the sensor unit, and includes a measurement result during one cycle of a sleep cycle rhythm of the user.

5. The sleeping environment control device of claim 1, wherein the current sleeping adequacy information is generated based on the current state information measured during one cycle of a sleep cycle rhythm.

6. The sleeping environment control device of claim 1, wherein the post-operation state information is generated based on a biometric signal during one cycle of a sleep cycle rhythm during which the operation unit performs an operation by a control signal of the processor.

7. The sleeping environment control device of claim 1, wherein the post-operation sleeping adequacy information is generated based on the post-operation state information measured during one cycle of a sleep cycle rhythm.

8. The sleeping environment control device of claim 1, wherein the reference sleeping adequacy information is generated based on a biometric signal that maximizes sleep efficiency of the user during one cycle of a sleep cycle rhythm and is a goal of the post-operation sleeping adequacy information.

9. The sleeping environment control device of claim 1, wherein the operation determination algorithm is formed of an artificial neural network, and outputs a score of each of one or more pieces of candidate operation information by using the current sleeping adequacy information as an input, and determines the operation information based on the score of each candidate operation information.

10. The sleeping environment control device of claim 1, wherein the operation adequacy determination module determines adequacy for the operation by comparing the post-operation sleeping adequacy information during one cycle of the sleep cycle rhythm with the reference sleeping adequacy information and determining similarity,
updates the operation determination algorithm so that a probability that the operation determination algorithm determines the operation information based on the current sleeping adequacy information increases when the similarity is high, or
updates the operation determination algorithm so that a probability that the operation determination algorithm determines the operation information based on the current sleeping adequacy information decreases when the similarity is low.

11. A method of controlling a sleeping environment by using reinforcement learning, the method comprising:
generating current state information by measuring a biometric signal of a user;
generating current sleeping adequacy information based on the current state information;
determining operation information by using an operation determination algorithm based on the current sleeping adequacy information;
performing an environment control operation of a mattress main body based on the operation information;
generating post-operation state information by measuring a biometric signal of the user after performing the environment control operation;
generating post-operation sleeping adequacy information based on the post-operation state information;
determining adequacy for the environment control operation by comparing the post-operation sleeping adequacy information and reference sleeping adequacy information; and
updating an operation determination algorithm based on the determination on the adequacy for the environment control operation.

12. A computer program which is executable by one or more processors and is stored in a computer readable medium, the computer program causing the one or more processors to perform following operations, the operations comprising:
generating current state information by measuring a biometric signal;
generating current sleeping adequacy information based on the current state information;
determining operation information by using an operation determination algorithm based on the current sleeping adequacy information;
performing an environment control operation of a mattress main body based on the operation information;
generating post-operation state information by measuring a biometric signal of the user after performing the environment control operation;
generating post-operation sleeping adequacy information based on the post-operation state information;
determining adequacy for the environment control operation by comparing the post-operation sleeping adequacy information and reference sleeping adequacy information; and
updating an operation determination algorithm based on the determination on the adequacy for the environment control operation.
